(19) Europäisches Patentamt
European Patent Office
Office européen des brevets

(11) Publication number: **0 073 145**
**A1**

# (12) EUROPEAN PATENT APPLICATION

(21) Application number: 82304385.6

(22) Date of filing: 19.08.82

(51) Int. Cl.³: G 03 C 7/34
//C07D213/75, C07D307/66, C07C127/19

(30) Priority: 20.08.81 JP 131313/81
20.08.81 JP 131314/81

(43) Date of publication of application:
02.03.83 Bulletin 83/9

(84) Designated Contracting States:
DE FR GB

(71) Applicant: KONISHIROKU PHOTO INDUSTRY CO. LTD.
No. 26-2, Nishishinjuku 1-chome Shinjuku-ku
Tokyo 160(JP)

(72) Inventor: Tsuda, Yasuo
3-3-5 HigashiGotanda
Shinagawa-ku Tokyo(JP)

(72) Inventor: Sato, Ryosuke
5995 Hino
Hino-shi Tokyo(JP)

(74) Representative: Ellis-Jones, Patrick George Armine et al,
J.A. KEMP & CO. 14 South Square Gray's Inn
London WC1R 5EU(GB)

## (54) A phenol cyan coupler for silver halide color photographic material.

(57) A phenol cyan coupler suitable for use in a silver halide color photographic material is *provided* comprising a phenol nucleus substituted by a group represented by the following Formula [I] or [II] at the 2-position and by an acylamino group at the 5-position:

Formula [I]

$$-NHCONH-R_1$$

wherein $R_1$ is a heterocyclic group or a condensed heterocyclic group,

Formula [II]

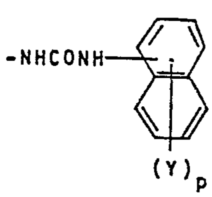

wherein Y is a monovalent group; p is o or an integer from one to five.

EP 0 073 145 A1

DESCRIPTION

"A PHENOL CYAN COUPLER FOR SILVER HALIDE COLOR PHOTOGRAPHIC MATERIAL"

The present invention relates to a novel coupler for forming a cyan dye image and also to a silver halide color photographic material containing said coupler. Normally, a color image is formed by a process in which an oxidation product of a color developing agent produced by reducing a silver halide grain that was exposed to light with an aromatic primary amine color developing agent and the couplers forming a yellow, magenta and cyan dye, respectively, undergo oxidative coupling with each other in a silver halide emulsion.

Couplers typically used for forming a cyan dye are those of a phenol or a naphthol. In a phenol, in particular, the fundamental properties heretofore required from the viewpoints of the photographic capability of the coupler, are that the spectral absorption characteristics of the dye should be very good, i.e. there should be no absorption but in a sharp green band in the spectrum of said dye; the dye formed should be fast to light, heat, dampness and the like; the color developability thereof should be very good, i.e. the dye should have satisfactory color sensitivity and color density; there should be no loss of dye even if a bleaching or bleach-fix solution

comprising principally EDTA ferric salt becomes exhausted.

From the viewpoint of environmental pollution prevention, benzyl alcohol should be eliminated from the color developing solution. It is, however, the case that satisfactory color developability cannot be attained, in the normal way, without adding benzyl alcohol. The lowering of color developability caused by the elimination of benzyl alcohol is serious particularly for a phenol cyan coupler, and therefore there is a demand for a highly color developable phenol cyan coupler requiring little or no benzyl alcohol for development.

For the purpose of satisfying the above-mentioned requirements, various studies have been conducted but so far, there has not been found any cyan coupler satisfying all the required properties.

For example 6-[α-(2,4-di-t-amylphenoxy)butanamide]-2,4-di-chloro-3-methyl phenol described in U.S. Patent 2,801,171 has excellent light resistance but is defective in its thermal resistance and besides the loss of color dye is considerable when a fatigued bleach-fix solution is used. It depends greatly on benzyl alcohol for color development dependence, so that it is difficult to eliminate benzyl alcohol from the color developing solution. 2-Heptafluorobutanamide-5-[α-(2,4-di-t-amylphenoxy)hexanamide]phenol, described in U.S. Patent 2,815,826 has excellent thermal resistance and the loss of

color dyes in a fatigued bleach-fix bath is slight but it has poor light resistance and color developability. Of the couplers described in Japanese Patent Publication Open to Public Inspection No. 109630/1978 (hereinafter referred to as Japanese Patent O.P.I. Publication), the elimination of benzyl alcohol still remains a problem as does their light resistance. Further, the phenol type cyan couplers described in U.S. Patent 3,839,044; Japanese Patent O.P.I. Publications 37425/1972, 10135/1975, 117422/1975, 130441/1975, 108841/1975 and 120334/1975; Japanese Patent Publication 36894/1973 and the like also possess unsatisfactory thermal resistance and developability in the absence of benzyl alcohol. With the phenol type couplers having an ureido group at the 2-position described in British Patent 1,011,940; U.S. Patents 3,446,622, 3,996,253, 3,758,308 and 3,880,661 and the like, the cyan dyes formed therefrom are not desirable from a color reproduction viewpoint, because their spectral absorption is broad and because the dyes each absorb considerably in the green band of the spectrum because there is an absorption maximum in the red band relatively close to the shortwave length area. The phenol couplers having an ureido group at the 2-position described in Japanese Patent O.P.I. Publication 65134/1981 are better as regards green absorption, but their other characteristics are not fully satisfactory.

We have now found, according to the present invention, a group of couplers which satisfy the various requirements for such phenol cyan couplers.

The present invention provides a phenol cyan coupler for silver halide color photographic material comprising a phenol nucleus substituted by a group represented by the following Formula [I] or Formula [II] at the 2-position and by an acylamino group at the 5-position. Formula [I]

$$-NHCONH-R_1$$

wherein $R_1$ is a heterocyclic group or a condensed hetero cyclic group;

Formula [II]

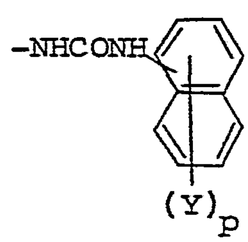

wherein Y is a monovalent group; p is o or an integer from one to five.

The cyan couplers of the present invention are preferably represented by the following Formula [III] or [IV]:

Formula [III]

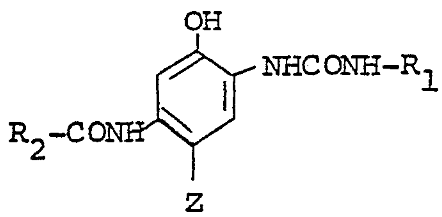

wherein $R_1$ represents a heterocyclic group or condensed hetero-cyclic group, preferably a five or six membered one, e.g. a furyl group, thienyl group, pyrrolyl group, pyridyl group, quinolyl group, pyrazinyl group, oxazolyl group, tetrazolyl group, benzothiazolyl group, benzofuranyl group or tetrahydrofuranyl group; $R_1$ may further be substituted, for example by an alkyl group having one to ten carbon atoms (such as an ethyl group, i-propyl group, i-butyl group, t-butyl group, or t-octyl group); an aryl group (such as a phenyl group or naphthyl group); a halogen atom (such as fluorine, chlorine or bromine); a cyano group; a nitro group; a sulfonamide group (such as a methanesulfonamide group, butane-sulfonamide group or p-toluene sulfonamide group); a sulfamoyl group (such as a methylsulfamoyl group or phenylsulfamoyl group); a sulfonyl group (such as a methanesulfonyl group or p-toluenesulfonyl group); fluorosulfonyl group; a carbamoyl group (such as a dimethylcarbamoyl group or a phenylcarbamoyl group); an oxycarbonyl group (such as an ethoxycarbonyl group or a phenoxycarbonyl group); a heterocyclic group (such as a

pyridyl group or pyrazolyl group); or other substituted group. The said substituents may be substituted independently; if two of them are used they may be the same or different, and these substituents may themselves be substituted by a substituent.

$R_2$ is a ballast group, preferably a straight-chain or branched alkyl group having 4 - 30 carbon atoms (such as a t-butyl group, n-octyl group, t-octyl group, or n-dodecyl group); an alkenyl group; an aralkyl group; an aralkenyl group; an alkoxyalkyl group; a substituted or unsubstituted cycloalkyl group; a five or six-membered heterocyclic group; or a group of the following Formula [V]:

Formula [V]

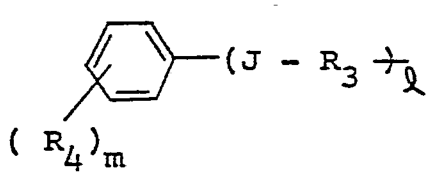

wherein J represents an oxygen atom or a sulfur atom; $R_3$ represents a straight-chain or branched alkylene group having 1 - 20 carbon atoms; $R_4$ represents an atom or group such as a hydrogen atom; a halogen atom (preferably a chlorine atom or bromine atom); an alkyl group [preferably, a straight chain or branched alkyl group having 1 - 20 carbon atoms (such as methyl, tert-butyl, tert-pentyl, tert-octyl, dodecyl or pentadecyl)]; an aryl group (such as phenyl); a heterocyclic group (preferably a nitrogen-containing heterocyclic group); an aralkyl group (such as

benzyl or phenethyl); an alkoxy group [preferably a straight-chain or branched alkyloxy group having 1 - 20 carbon atoms (such as methoxy, ethoxy, tert-butyloxy, octyloxy, decyloxy or dodecyloxy)]; an aryloxy group (such as phenoxy); a hydroxy group; an acyloxy group [preferably a substituted or unsubstituted alkylcarbonyloxy group or an arylcarbonyloxy group (such as acetoxy or benzoyloxy)]; a carboxy group; an alkoxycarbonyl group (preferably a substituted or unsubstituted straight-chain or branched alkyloxy carbonyl group having 1 - 20 carbon atoms); an aryloxy carbonyl group (preferably substituted or unsubstituted phenoxy carbonyl); a mercapto group; an alkylthio group (preferably substituted or unsubstituted straight-chain or branched benzenesulfonyl having 1 - 20 carbon atoms); an acyl group (preferably straight-chain or branched alkylcarbonyl); an acylamino group (preferably straight-chain or branched alkylcarbonamido having 1 - 20 carbon atoms or substituted or unsubstituted benzenecarbonamido); a sulfonamide group (preferably a straight-chain or branched, substituted or unsubstituted alkyl sulfonamide group having 1 - 20 carbon atoms, or a substituted or unsubstituted benzene sulfonamide group); a carbamoyl group (preferably straight-chain or branched alkylaminocarbonyl having 1 - 20 carbon atoms; or substituted or unsubstituted phenylamino carbonyl); and a sulfamoyl group (preferably straight-chain or branched

alkylamino sulfonyl, or substituted or unsubstituted phenylamino sulfonyl); Z represents a group which is eliminatable at the time of coupling to the oxidation product of hydrogen or a color developing agent e.g. a halogen atom such as a chlorine, bromine or fluorine atom; an aryloxy group, carbamoyloxy group, carbamoylmethoxy group, acyloxy group, sulfonamide group, or succinimide group, an oxygen atom or nitrogen atom thereof being coupled directly at the coupling position; concrete examples are given in U.S. Patent No. 3,741,563; Japanese Patent O.P.I. Publication No. 37425/1972, 10135/1975, 117422/1975, 130441/1975, 108841/1975, 120334/1975, 18315/1977, 52423/1978 and 105226/1978; and Japanese Patent Examined Publication No. 36894/1973; m has an integral value from one to four; and l is zero or one.

Formula [IV]

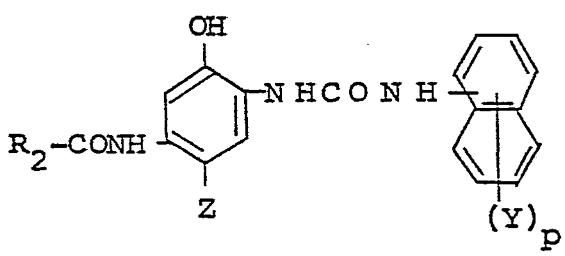

wherein Y represents a monovalent group, for example a substituted or unsubstituted alkyl group [preferably alkyl having one to four carbon atoms such as methyl or tert-butyl]; an aryl group [preferably a substituted or unsubstituted phenyl group]; a heterocyclic group [preferably a nitrogen-containing hetero-ring such as

pyrrolidine or piperidine]; a hydroxy group; an alkoxy group [preferably a substituted or unsubstituted alkoxy group having one to eight carbon atoms such as methoxy-tert-butyloxy group or methoxycarbonyl methoxy group]; an aryloxy group [preferably a substituted or unsubstituted phenoxy group]; an acyloxy group [preferably a substituted or unsubstituted alkyl carbonyloxy group or an aryl carbonyloxy group]; a mercapto group; an alkylthio group [preferably a substituted or unsubstituted alkylthio group having one to eight carbon atoms such as a methylthio group]; a nitro group; an acyl group [preferably an alkyl carbonyl group such as an acetyl group or a pivaloyl group]; an amino group; an alkylamino group [preferably a straight-chain or branched alkylamino group having one to four carbon atoms such as a methylamino group, an ethylamino group or tert-butyl amino group]; a dialkylamino group such as a dimethylamino group or diethylamino group; a halogen atom such as a chlorine atom or bromine atom; a cyano group; or a group of formula $-COOR'$, $-CON(R')_2$, $-SO_2R$, $-SO_2OR'$, $-SO_2N(R')_2$, or $-SOR$, [wherein R represents a substituted or unsubstituted alkyl, alkenyl, cycloalkyl, aryl or heterocyclic group; and R' represents a hydrogen atom or a group represented by said R, each R' being identical or different, and both may form together a five- or six-membered ring with the nitrogen atom, for example a methane sulfonyl group,

carbamoyl group, methane sulfinyl group, ethoxy carbonyl group, isopropyl sulfamoyl group or methoxy sulfonyl group].

$R_2$ represents a ballast group as defined in Formula [III].

Z represents a hydrogen atom or a group capable of eliminating at the moment of coupling with the oxidant of a color developing agent, as defined in Formula [III].

P represents 0 or an integer from 1 to 5.

The cyan couplers of this invention include the following as illustrative:

(1)

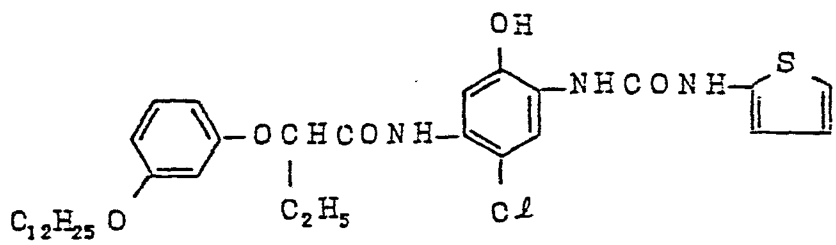

(2)

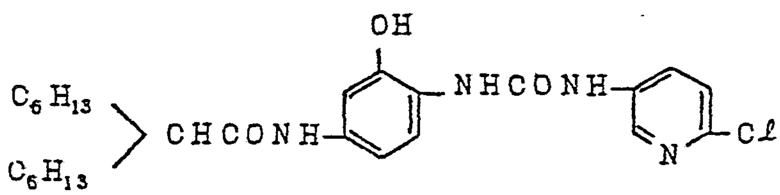

(3)

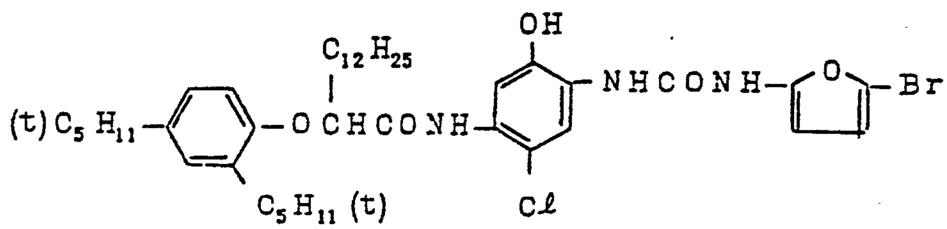

(4)

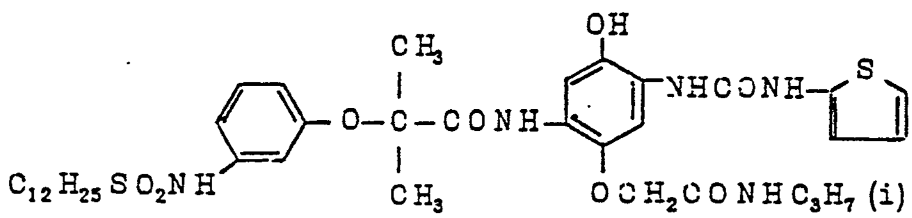

(5)

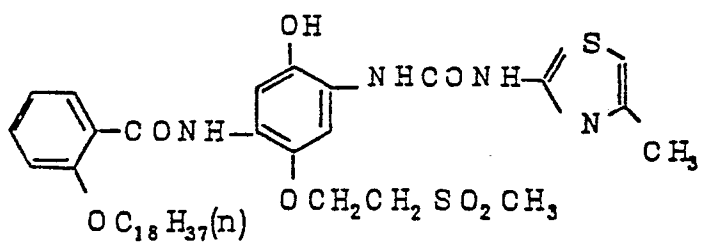

(6)

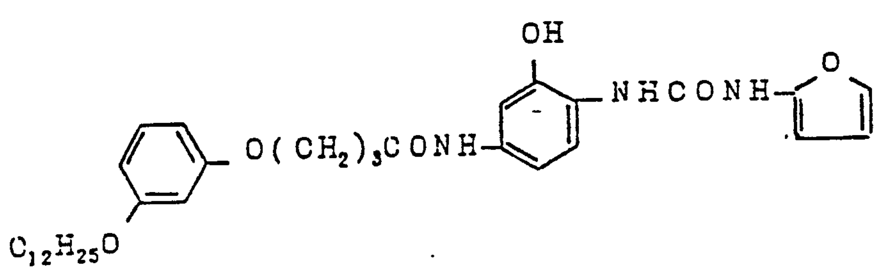

(7)

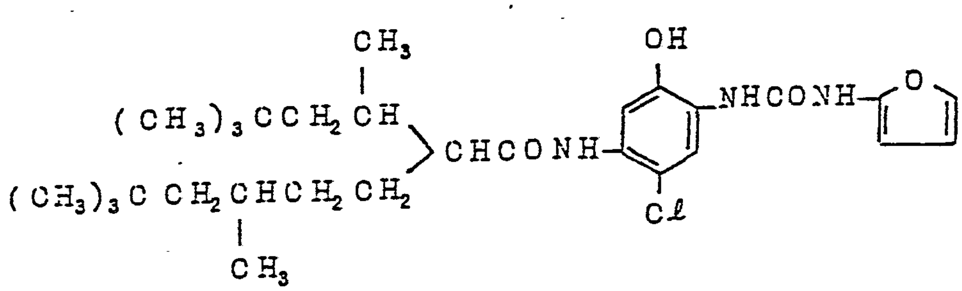

(8)

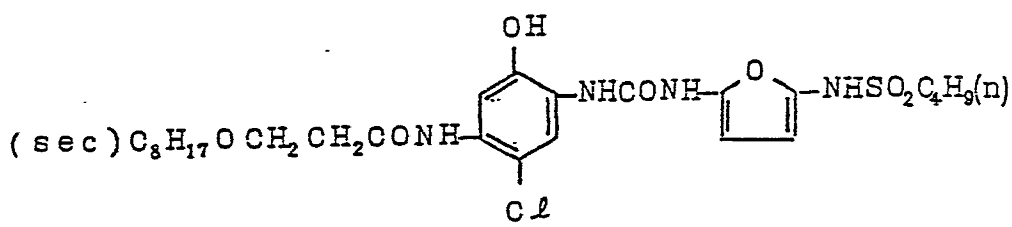

(9)

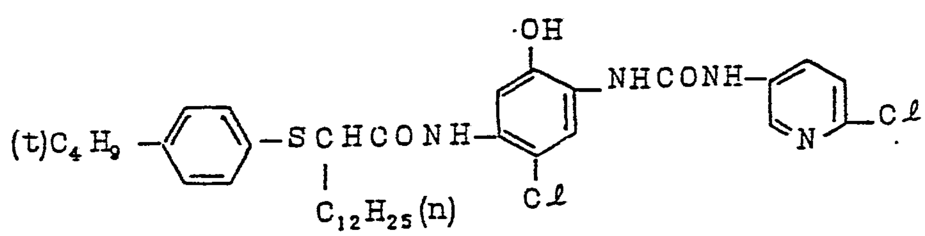

(10)

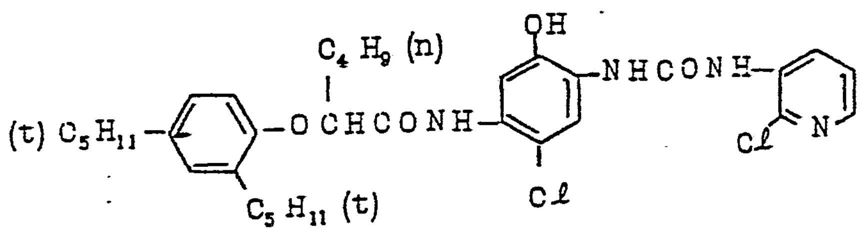

(11)

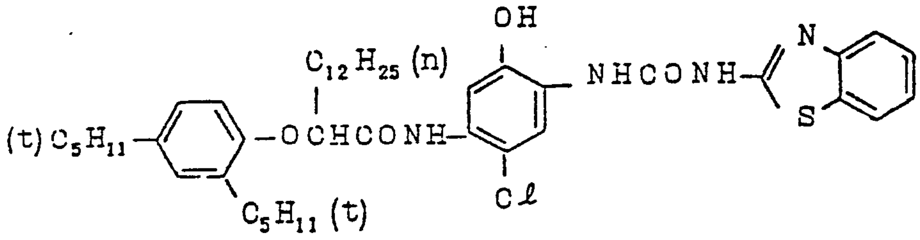

(12)

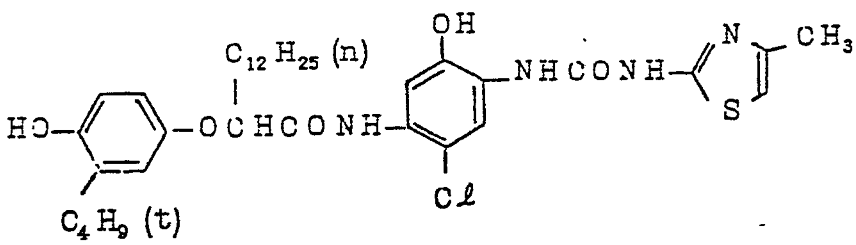

(13)

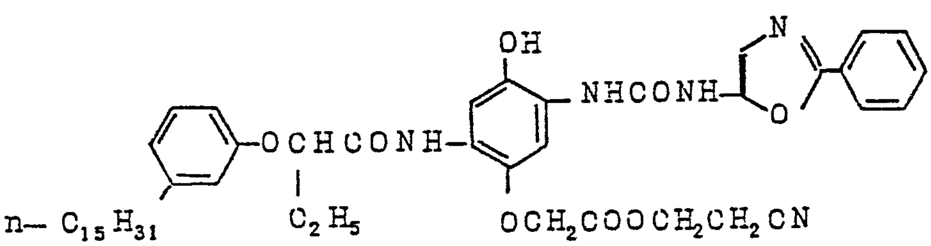

(14)

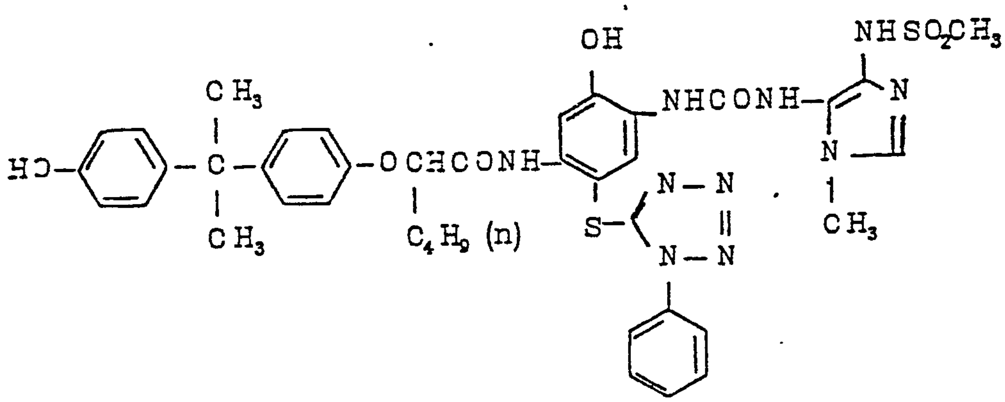

(15)

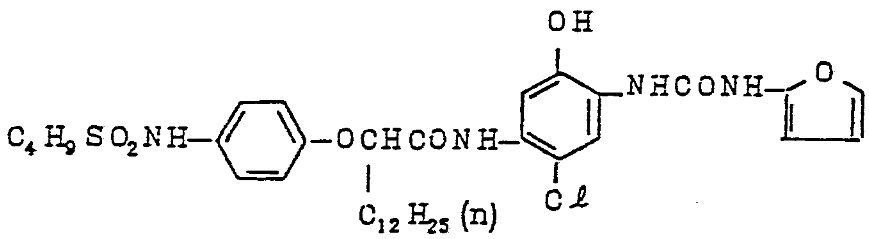

(16)

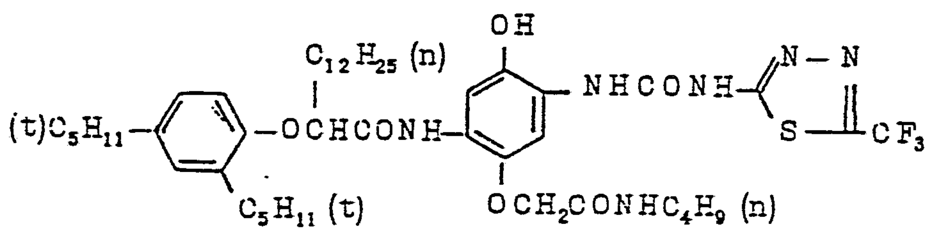

(17)

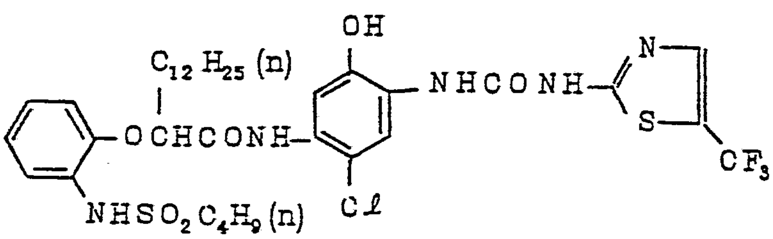

(18)

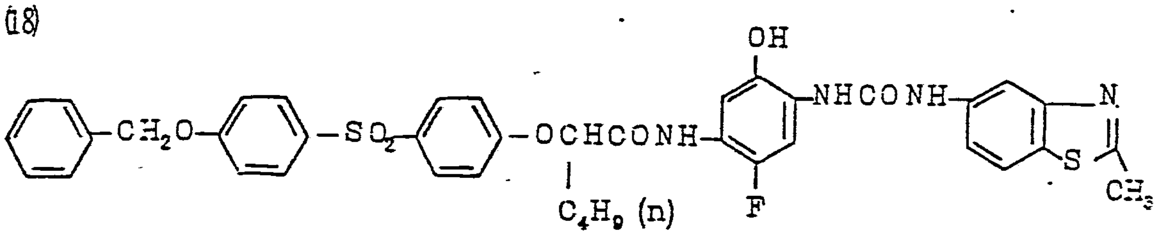

(19)

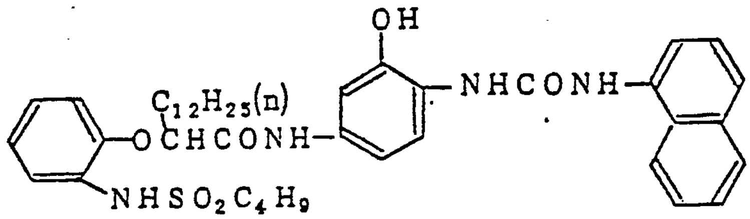

(20)

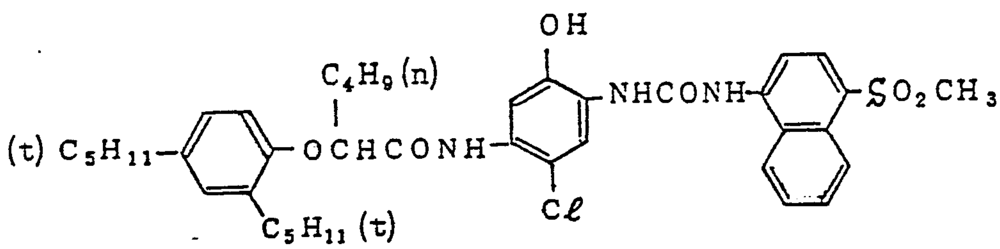

(21)

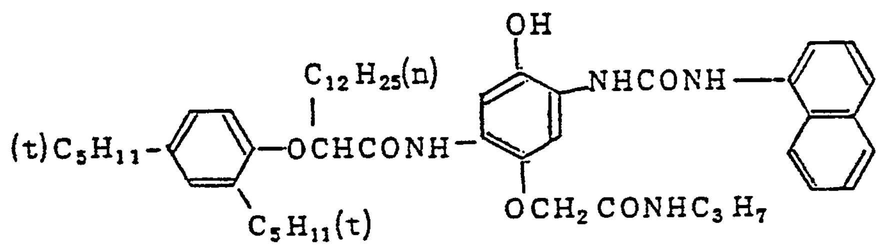

(22)

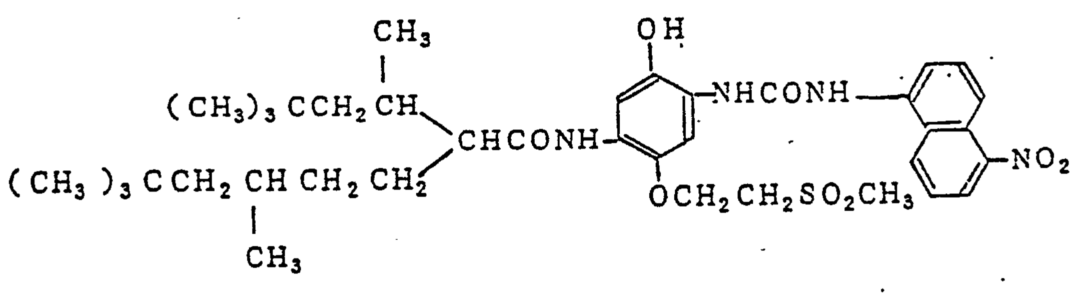

(23)

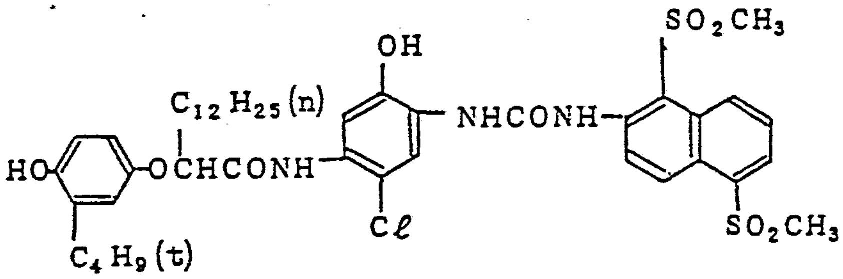

(24)

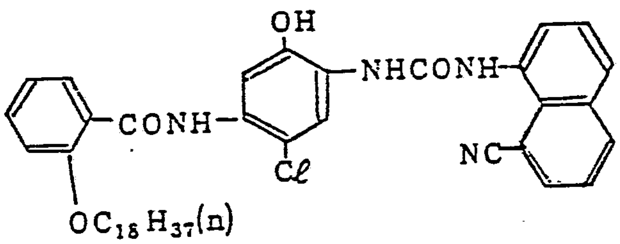

(25)

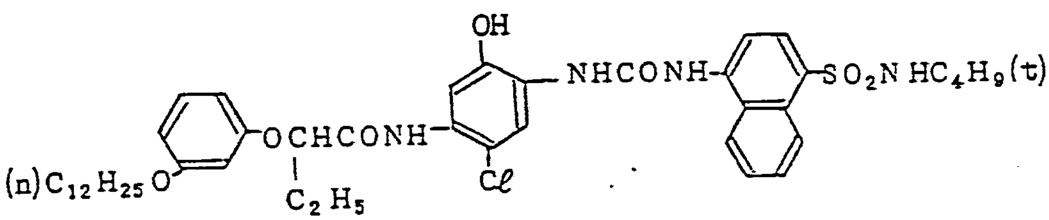

(26)

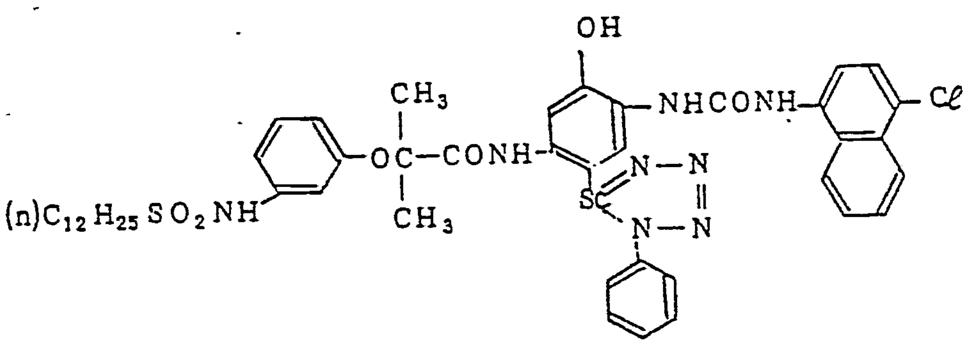

(27)

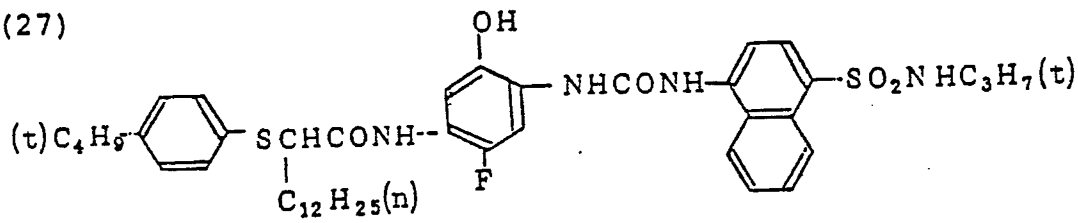

(28)

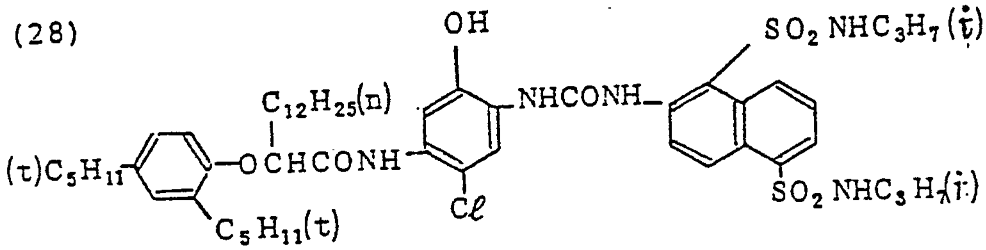

(29)

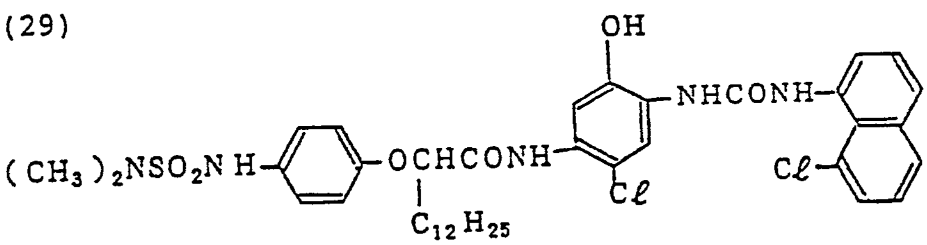

(30)

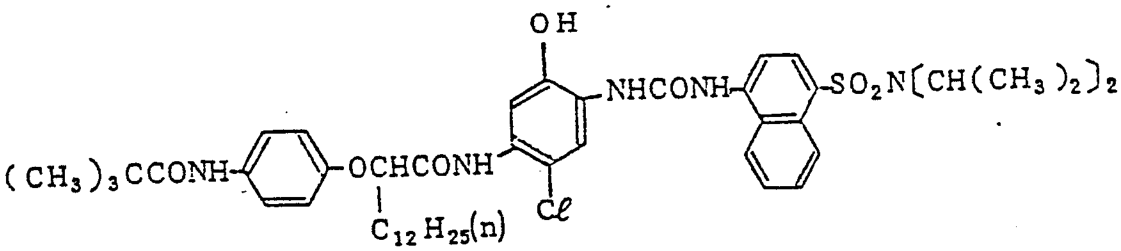

(31)

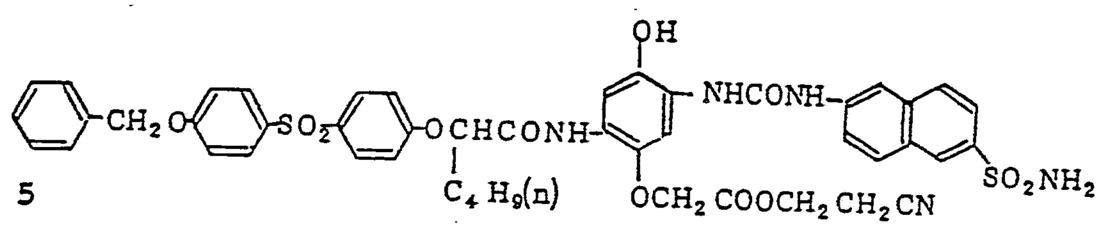

(32)

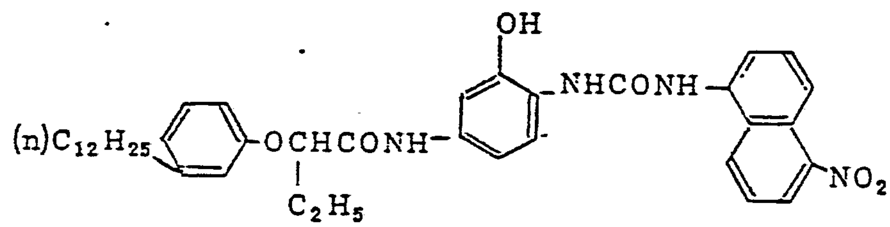

(33)

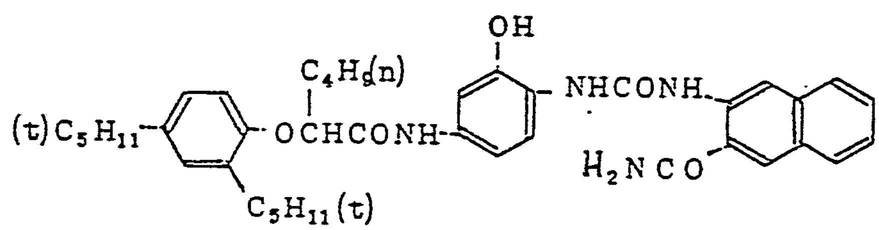

(34)

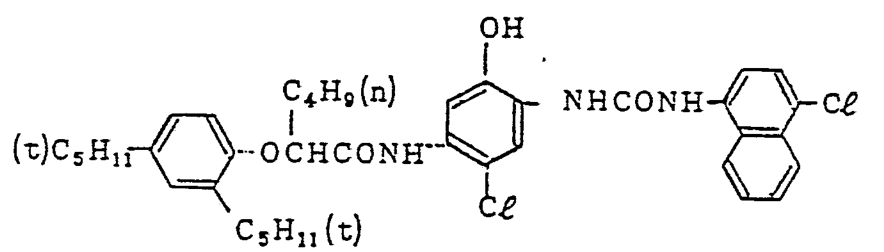

(35)

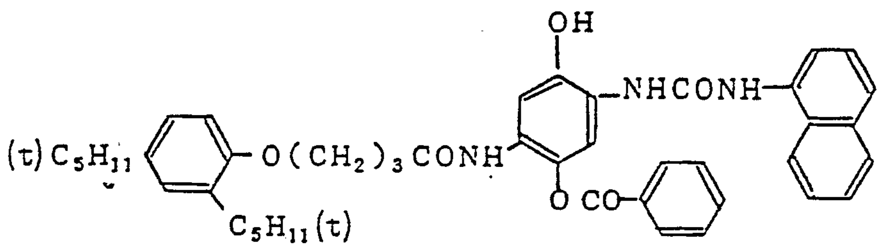

(36)

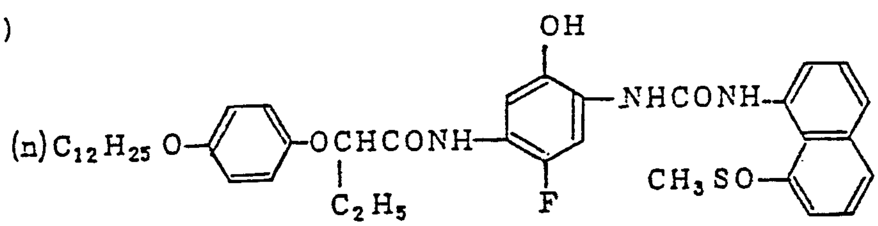

(37)

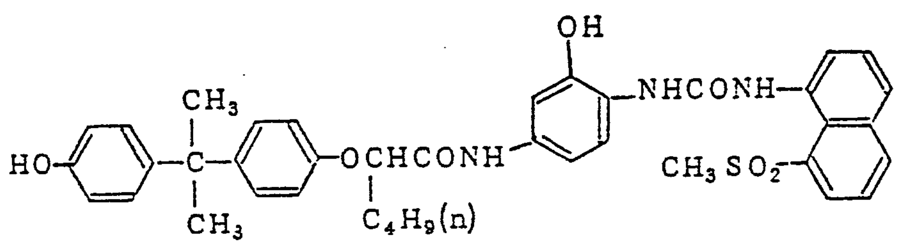

(38)

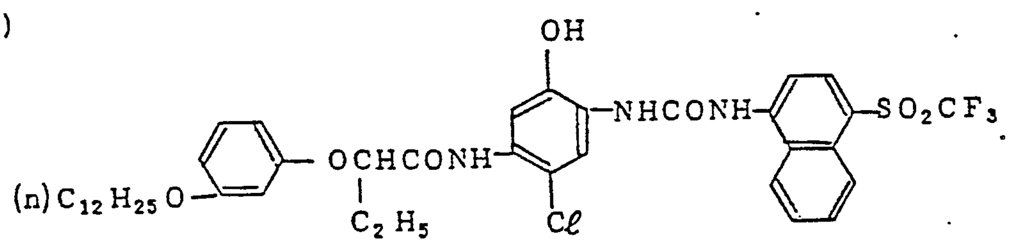

(39)

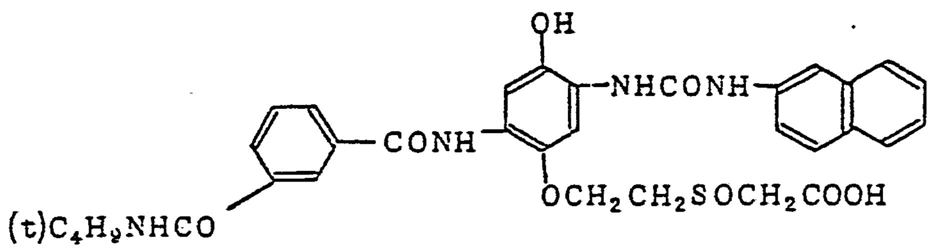

(40)

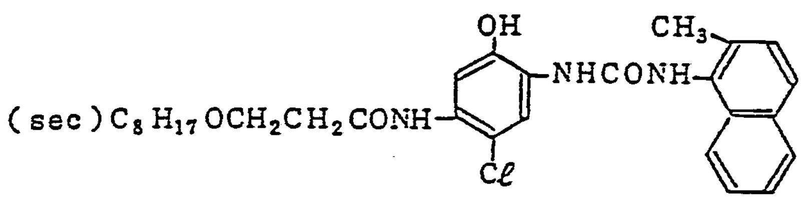

(41)

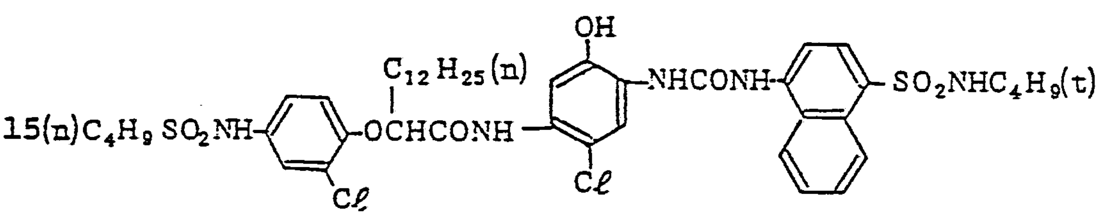

(42)

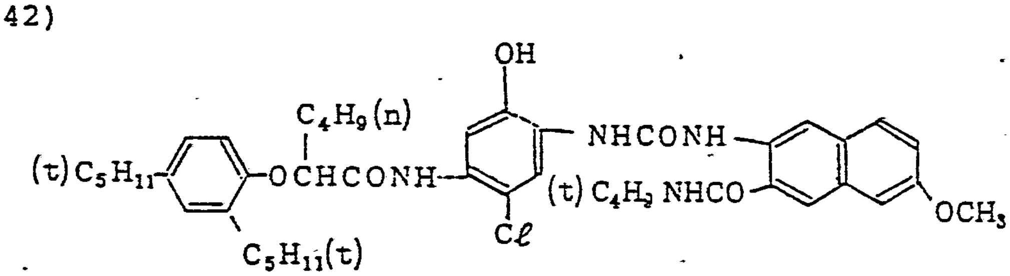

Typical routes for synthesizing the couplers of the invention and the synthesis Examples thereof are given below.

(1)

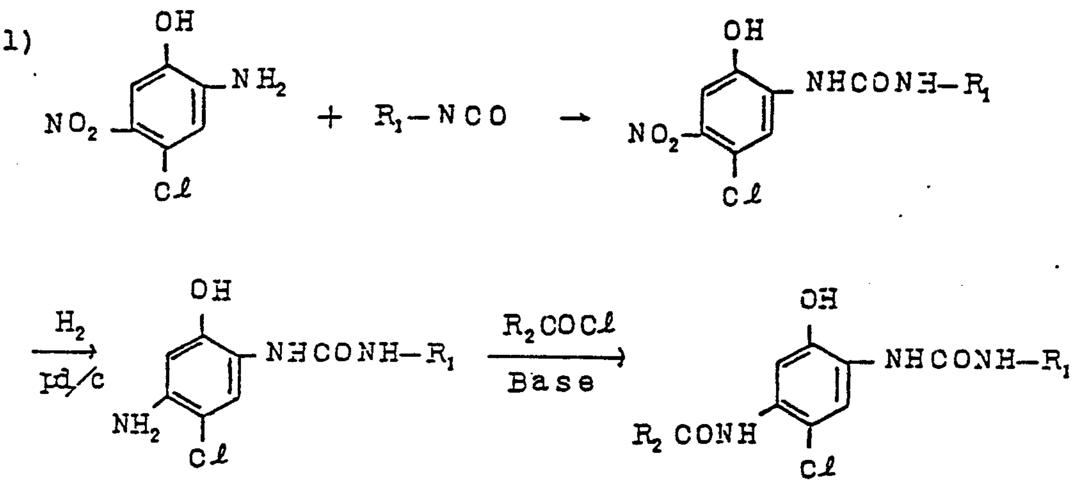


(2)

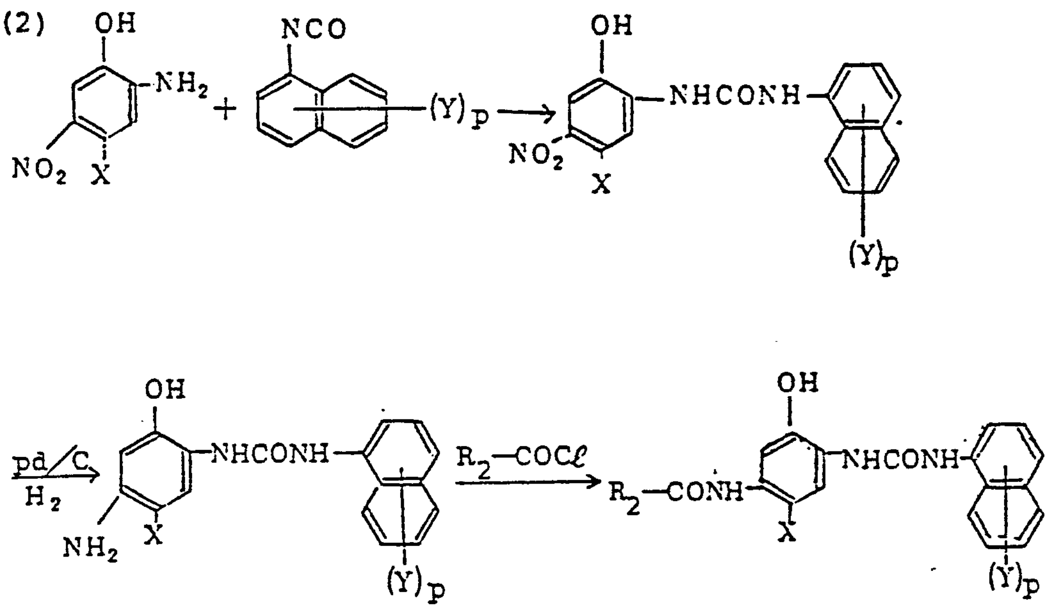

Synthesis Example 1 (synthesis of Exemplified Coupler 10)

1) Synthesis of 2-(2-chloro-3-pyridyl)ureido-4-chloro-5-nitrophenol:

The suspension of 18.9 g of 2-amino-4-chloro-5-nitrophenol was made in 200 ml of toluene, and the suspension thus obtained was added with the solution prepared by dissolving 15.5 g of 2-chloro-3-pyridyl isocyanate into 100 ml of toluene at room temperature and with agitation, and the reflux was further applied thereto for two hours. The crystals which were produced by cooling at room temperature after the reflux, were filtrated and washed with toluene and methanol in succession, and were then dried up.

The object in light yellow-brown in 31.7 g were thus obtained.

2) Synthesis of Exemplified Coupler 10:

The dissolution of 6.7 g of 2-(2-chloro-3-pyridyl)ureido-4-chloro-5-nitrophenol was made with 500 ml of tetrahydrofuran and the catalytic reduction was carried out with palladium-carbon to serve as the catalizer. After the theoretical amount of hydrogen was absorbed from the reductant, the catalizer was filtrated. The filtrate thus obtained was then vacuum-concentrated. The residuum thus obtained was added with 200 ml of acetonitrile and 2.0 ml of pyridine, and the solution thus obtained was added gradually with the solution prepared by dissolving 7.3 g of 2-(2,4-di-tert-pentylphenoxy)hexanoyl chloride

into 50 ml of acetonitrile at room temperature and with agitation, and the agitation was kept on further for six hours. The reaction liquid was added into ice water and the oily matters separated therefrom were extracted by applying ethyl acetate. Then the vacuum-concentration was carried out after the dehydration was done with magnesium sulfate. The crude products thus obtained were separated by means of silica gel column chromatography. The products thus separated were dissolved with ethyl acetate and n-hexane and were then caked, and the recrystallization was further made with methanol. Thus, the object in white crystal of 3.8 g were obtained. The structure thereof was confirmed by the mass spectrograph and the nuclear magnetic resonance spectrograph.

Synthesis Example 2 (Synthesis of Exemplified Coupler 15)

1) Synthesis of 2-(2-furyl)ureido-4-chloro-5-nitrophenol:

The suspension of 9.4 g of 2-amino-4-chloro-5-nitrophenol was made in 150 ml of toluene and the suspension thus obtained was added gradually with the solution prepared by dissolving 5.5 g of furyl isocyanate into 50 ml of toluene at the raised temperature up to 40°C and with agitation, and the agitation was further kept on at 80°C for four hours. The reaction liquid thus obtained was cooled with water and the crystals thereby produced were filtrated and washed sufficiently with toluene and methanol in succession, and were then dried up.

Thus, the object in light yellow-brown of 11.8 g were obtained.

2) Synthesis of Exemplified Coupler 15:

The dissolution of 6.0 g of 2-(2-furyl)ureido-4-chloro-5-nitrophenol was made with 500 ml of tetrahydrofuran and the catalytic reduction was made with palladium-carbon to serve as the catalizer. After the theoretical amount of hydrogen was absorbed from the reductant, the catalizer was filtrated. The filtrate thus obtained was then vacuum-concentrated. The amino substance thus obtained was mixed without refining with 200 ml of acetonitrile and 2.0 ml of pyridine, and was then added gradually with the solution prepared by dissolving 9.5 g of 2-(4-butansulfonylaminophenoxy)tetradecanoyl chloride into 50 ml of acetonitrile, and the agitation was further kept on for three hours. The reaction liquid was added in the ice-water containing a small amount of chloric acid and then the oily matters which were separated therefrom was extracted by applying ethyl acetate. Then, the vacuum-concentration was carried out after the dehydration was done with magnesium sulfate, and thus, the crude products in the form of a brown tar were obtained. Said crude products were separated by making use of toluene-ethyl acetate to serve as the elute through the silica gel column chromatography. The products thus separated were dissolved with ethyl acetate and n-hexane to make them precipitate, and thus, the object in the form of white fine powders of 3.3 g were obtained. The structure thereof was confirmed by

the mass spectrograph and the nuclear magnetic resonance spectrograph.

Synthesis Example 3 (Synthesis of Exemplified Coupler 19)

1) Synthesis of 2-naphthyl ureido-5-nitrophenol

2-amino-5-nitrophenol of 15.4 g were suspended in 500 ml of toluene, and 16.9 g of naphthyl isocyanate were dropped into the suspension thus prepared spending an hour with the heating at 80°C and the agitation. After the dropping thereof was completed, the further reaction was carried out for another two hours with the reflux. Thus obtained reaction solution was cooled down to room temperature and the precipitate thus produced was filtrated and was washed with toluene and n-hexane in succession, and was then dried up. Thus, 30.6 g of light yellow-brown powders were obtained.

2) Synthesis of Exemplified Coupler 19

The dissolution of 6.5 g of 2-naphthyl ureido-5-nitrophenol was made with 300 ml of tetrahydrofuran, and the catalytic reduction was carried out with 1 g of palladium-carbon to serve as the catalizer. After the theoretical amount of hydrogen was absorbed, the catalizer was swiftly filtrated and 4 ml of pyridine were added into the filtrated solution, and then the solution of 8.1 g of 2-(2-nitrophenoxy) tetradecanoyl chloride and 50 ml of tetrahydrofuran was dropped thereinto spending thirty minutes with cooling with ice-water.

The reaction liquid was agitated for another two hours at room temperature, and said agitated liquid was then added into one liter of ice-water containing 10 ml of chloric acid and the extraction was made with ethyl sulfate and the dehydration was made with magnesium sulfate; and then the vacuum-concentration was carried out. The crude products thus obtained was separated and refined by means of silica gel column chromatography.

The catalytic reduction was carried out in the process that 6.0 g of the isolated nitro-substance were dissolved in 100 ml of tetrahydrofuran and one gram of palladium-carbon was used to serve as the catalizer. After the theoretical amount of hydrogen was absorbed, the catalizer was filtrated. The filtrate thus obtained was then vacuum-concentrated. The residuum thus obtained was added with 100 ml of acetonitrile and 1.0 ml of pyridine, and 1.6 g of butane sulfonyl chloride were dropped thereto spending thirty minutes at room temperature and with agitation, and the further reaction was kept on for another two hours. Thus obtained reaction liquid was vacuum-concentrated and dissolved in ethyl acetate, and then dried up. The dehydration was made with magnesium sulfate and the vacuum concentration was carried out, and thus obtained crude product was separated and refined with silica gel column chromatography. The crystallization was made with the mixed solution of ethyl sulfate and n-hexane, and thus, 3.2 g of white powders were obtained. It was confirmed by the analysis

through the nuclear magnetic resonance spectrography and the mass spectrography that the obtained was the substance of the object.

Synthesis Example 4 (Synthesis of Exemplified Coupler 34)

1. Synthesis of 2-(4-chloro-1-naphthyl)ureido-4-chloro-5-nitrophenol

The suspension of 18.9 g of 2-amino-4-chloro-5-nitrophenol, 29.8 g of 4-chloro-1-phenyl naphthylcarbamate and 1.5 g of imidazole was suspended in 250 ml of xylene and the agitation reaction was then carried out for seven hours with reflux. The reaction liquid was cooled down to room temperature and the precipitate thus produced was filtrated, the filtrate was washed with xylene and n-hexane in succession, and thus the drying thereof was made. Thus, 37.7 g of light yellow-brown powders were obtained.

2. Synthesis of Exemplified Coupler 34

The dissolution of 7.8 g of 2-(4-chloro-1-naphthyl)ureido-4-chloro-5-nitrophenol was made into 300 ml of tetrahydrofuran, and then the catalytic reduction thereof was carried out with one gram of palladium-carbon to serve as the catalizer. After the theoretical amount of hydrogen was absorbed, the catalizer was swiftly filtrated, and 4 ml of pyridine was added into thus obtained filtrated liquid, and then 7.3 g of 2-(2,4-di-tert-pentyl phenoxy)hexanoyl chloride were dropped therein

spending thirty minutes with cooling with ice-water. The reaction liquid was agitated for another two hours at room temperature, and said agitated liquid was then added into one liter of ice-water containing 10 ml of chloric acid and the extraction was made with ethyl sulfate and the dehydration was made with magnesium sulfate; and then the vacuum-concentration was carried out. The crude products thus obtained was separated and refined by means of silica gel column chromatography. Thus refined product was solidified by making use of the mixed solution of ethyl acetate and n-hexane, and thus, 5.1 g of white powderd crystals were obtained. It was confirmed by the analysis through the nuclear magnetic resonance spectrography and the mass spectrography that the obtained was the substance of the object.

To the cyan dye forming couplers of this invention, any process or technique for the ordinary types of cyan dye forming couplers can similarly be applied. Typically, a photographic element is formed by incorporating the coupler into a silver halide emulsion and said emulsion is then coated over a substrate.

Such a photographic element may be a monochromatic or a multichromatic element. Said multichromatic element has a non-sensitizing emulsion or a color dye image forming constituent unit which is sensitive respectively to the area of each of the three primary colors in the spectrum, though the

cyan dye forming couplers of the invention are usually held in a red-sensitive emulsion; each of said constituent units may be composed of a single emulsion layer or a multi-coated emulsion layer which is sensitive to a certain area of the spectrum. As is well known by those skilled in the art, the layers of said elements including said image forming constituent units can be arranged in various orders. A typical multichromatic photographic element comprises a cyan dye image forming constituent unit comprising at least one red-sensitive silver halide emulsion layer having at least one cyan dye forming coupler (at least one of said cyan dye forming couplers being a coupler of the invention) and a yellow image forming constituent unit comprising at least one blue-sensitive silver halide emulsion layer having at least one magenta dye forming coupler, supported on a substrate. Said element can be provided with additional layers such as a filter layer, an inter-layer, a protective layer and a subbing layer.

In order to incorporate the couplers of the invention in an emulsion conventional processes can be used. For example, a silver halide emulsion to be used in the invention can be prepared by using an organic solvent having a boiling point of no lower than 175°C such as tricresylphosphate and dibutylphthalate or a solvent having a low boiling point such as butyl

acetate and butyl propionate, or a mixed solution thereof as occasion demands, to dissolve the couplers of the invention independently or together, and the solution thus obtained is mixed with an aqueous gelatin solution containing a surface active agent; emulsification can be carried out by means of a high speed rotary mixer or a colloid mill, and then a silver halide is added to the thus emulsified matter. When the couplers of the invention are to be added to a silver halide emulsion, the amount of said coupler is typically 0.07 to 0.7 mol, more preferably 0.1 to 0.4 mol, per mol of silver halide.

The silver halide to be used in a silver halide emulsion of the invention includes any silver halide used in an ordinary silver halide emulsion, for example silver bromide, silver chloride, silver iodobromide, silver chlorobromide or silver chloriodobromide.

Silver halide emulsions constituting a silver halide emulsion of the invention can be prepared by a variety of processes including those usually carried out, including, for example, the process as described in Japanese Patent Examined Publication No. 7772/1971, wherein an emulsion of silver salt grains comprising at least one part of silver salts whose solubility is greater than that of silver bromide is prepared, and then, at least one part of said grains is converted into silver bromide or silver iodobromide, that is, the so-called

conversion emulsion, or a process for preparing a Lippmann emulsion comprising a fine grain silver halide having an average grain diameter not larger than 0.1 μ.

Silver halide emulsions of the invention can be chemically sensitized by making use of one or more of a sulphur sensitizer such as allylthiocarbazide, thiourea or cystine; an active or inactive selenium sensitizer; a reduction sensitizer such as stannour salt or polyamine; a noble-metal sensitizer such as a gold sensitizer, more specifically potassium aurithiocyanate, potassium chloroaurate or 2-auro-sulfobenzthiazole methyl chloride; or a water-soluble salt sensitizer such as a ruthenium salt, rhodium salt or iridium salt, and more specifically ammonium chloropalladate, potassium chloroplatinate or sodium chloropalladide.

Silver halide emulsions of the invention may contain a variety of known photographic additives. For example, the photographic additives described in "Research Disclosure", Dec., 1978, Article 17643 can be used.

For the purpose of providing sensitiveness to a photosensitive wavelength range necessary for a red-sensitive emulsion, the silver halide of the invention can be spectrally sensitized by selecting suitable sensitizing dyes. A variety of spectrally sensitizing dyes can be used and more than one can be used.

In the invention, the spectrally sensitizing

dyes used advantageously are typically cyanine dyes, merocyanine dyes or complex cyanine dyes, as described in, for example, U.S. Patent Nos. 2,269,234, 2,270,378, 2,442,710, 2,454,520 and 2,776,280.

Color developing solutions which can be used in the present invention are preferably those having an aromatic primary amine color developing agent as the principal component. Concrete examples of said color developing agents include those of the p-phenylene diamine type, inter alia, diethyl-p-phenylenediamine chloride, monomethyl-p-phenylene diamine chloride, dimethyl-p-phenylene diamine chloride, 2-amino-5-diethylamino toluene chloride, 2-amino-5-(N-ethyl-N-dodecylamino)-toluene, 2-amino-5-(N-ethyl-N-β-methane sulfonamide ethyl)amino toluene chloride, 4-(N-ethyl-N-β-methane sulfonamide ethylamino)aniline, 4-(N-ethyl-N-β-hydroxy ethylamino)-aniline and 2-amino-5-(N-ethyl-N-β-methoxy ethyl)amino toluene.

After developing, the processes ordinarily carried out in succession for bleaching, fixing or bleaching-fixing, washing and drying, in order to remove silver and silver halide, can be used.

The following Examples further illustrate the present invention.

Example (1)

There were taken 0.03 mol each of the couplers of the invention indicated in Table 1 and the undermentioned control couplers, (A), (B) and (C) to add respectively into each of the mixed solutions of dibutyl phthalate whose amount was equivalent to the weight of said coupler added and ethyl acetate whose amount was three times as much as the weight of said coupler added, and then the dissolution was completed by raising the temperature up to 60°C. And each of the coupler dispersion solution was prepared in the process that the solution thus obtained was added into the aqueous solution of Alkanol B (alkylnaphthalene sulfonate - mfd. by Du Pont) and gelatin, and the emulsification was made by means of a colloid-mill. Next, each of said coupler dispersion solutions was added into silver chlorobromide emulsion of 0.1 mol in silver equivalent (20 mol% thereof were silver bromide) and thus obtained solution was coated on a sheet of polyethylene laminated paper and was then dried up, and thus, six kinds, in all, of the silver halide color photographic materials (Sample No. [1] - [6]) having the stably coated layer were obtained, respectively.

Control Coupler [A]

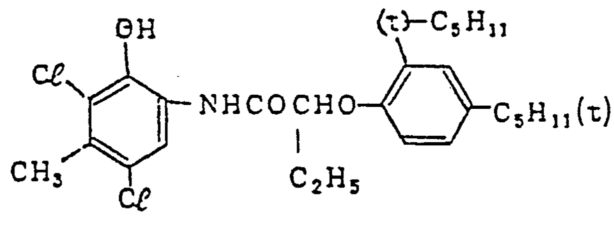

Control Coupler [B]

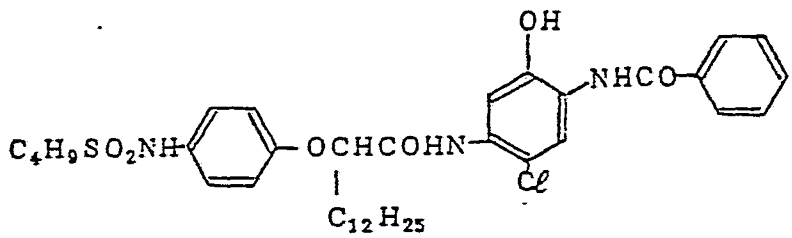

Control Coupler [C]

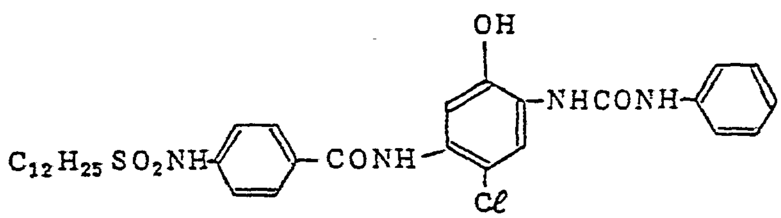

Each of these samples was exposed to light through a wedge and then processed as mentioned hereunder, provided that the color developing processes were carried out respectively on the two kinds of the compositions, i.e.; the composite added with benzyl alcohol (Color development [1]) and the composite without addition thereof (Color development [2]).

[Process]

| Processing Steps (at 30°C) | Processing time |
|---|---|
| Color development | 3' 30" |
| Bleach-Fix | 1' 30" |
| Wash | 2' |

The following shows each of the processing compositions:

[Composition of Color Developing Solution 1]

| | |
|---|---|
| 4-amino-3-methyl-N-ethyl-N-(β-methane sulfonamide ethyl)-aniline sulfate | 5.0 g |
| Benzyl alcohol | 15.0 ml |
| Sodium hexametaphosphate | 2.5 g |
| Sodium sulfite, anhydrous | 1.85 g |
| Sodium bromide | 1.4 g |
| Potassium bromide | 0.5 g |
| Borax | 39.1 g |
| Add water to make | 1.0 ltr. |
| Adjust pH value by sodium hydroxide to pH 10.30 | |

[Composition of Color Developing Solution 2]

| | |
|---|---|
| 4-amino-3-methyl-N-ethyl-N-(β-methane sulfonamide ethyl)-aniline sulfate | 5.0 g |
| Sodium hexametaphosphate | 2.5 g |
| Sodium sulfite, anhydrous | 1.85 g |
| Sodium bromide | 1.4 g |
| Potassium bromide | 0.5 g |
| Borax | 39.1 g |
| Add water to make | 1.0 ltr. |
| Adjust pH value by sodium hydroxide to pH 10.30 | |

[Composition of Bleach-fix Solution]

| | |
|---|---|
| Iron ethylenediamine tetraacetate ammonium salt | 50 g |
| Ammonium sulfite (40% solution) | 50 ml |
| Ammonium thiosulfate (70% solution) | 140 ml |

| | |
|---|---|
| Aqueous ammonia (28% solution) | 20 ml |
| Ethylenediamine tetraacetate | 4 g |
| Add water to make | 1 ltr. |

The photographic characteristics of each sample thus obtained were measured. The results thereof are tabulated in Table 1.

Wherein, the values of the relative sensitivity are indicated relatively to the maximum value of sensitivity, that is regarded as the value of 100, obtained when the processing was made with Color developing solution (1)

Table 1

| Sample No. | Coupler used | Color Development (1) | | Color Development (2) | |
|---|---|---|---|---|---|
| | | Relative Sensitivity | Max. Density | Relative Sensitivity | Max. Density |
| 1 | Exemplified Compound - 1 | 97 | 2.24 | 74 | 1.80 |
| 2 | " - 3 | 100 | 2.28 | 76 | 1.85 |
| 3 | " - 8 | 98 | 2.19 | 72 | 1.74 |
| 4 | Control Coupler - A | 97 | 2.17 | 51 | 1.45 |
| 5 | " - B | 93 | 1.90 | 64 | 1.49 |
| 6 | " - C | 85 | 1.82 | 55 | 1.47 |

It is obvious from the above Table 1, it is found that the samples obtained by using the couplers of the invention are capable of obtaining the excellent sensitivity and maximum density with or without addition of benzyl alcohol.

Also, as the result of measuring the chromatic emission spectrum, it was found that the dyes having used the couplers of the invention had the absorption maximum in the portion of the relatively longer wavelength in the red area and had a less absorption on the short wavelength side, so that an excellent color purity displayed.

Example (2)

The light resistance, heat resistance and moisture resistance of a color dye image were studied on the samples prepared in the similar process to that taken in Example (1).

The results therefrom are tabulated in Table 2.

Table 2

| Sample No. | Coupler used | Color Development (1) | | | Color Development (2) | | |
|---|---|---|---|---|---|---|---|
| | | Light Re-sist-ance | Heat Re-sist-ance | Moisture Resist-ance | Light Re-sist-ance | Heat Re-sist-ance | Moisture Resist-ance |
| 7 | Exemplified Compound - 1 | 87 | 96 | 98 | 86 | 96 | 98 |
| 8 | " - 3 | 90 | 98 | 97 | 88 | 98 | 96 |
| 9 | " - 8 | 85 | 96 | 94 | 83 | 97 | 96 |
| 10 | Control Coupler - A | 85 | 45 | 60 | 86 | 46 | 62 |
| 11 | " - B | 62 | 95 | 93 | 57 | 95 | 95 |
| 12 | " - C | 55 | 92 | 92 | 52 | 94 | 93 |

In the above Table 2, the value of the light resistance of each coupler is indicated by the relative value of the residual density of each image exposed by a xenon fade meter for 300 hours to the value of the density thereof measured before the exposure that is regarded as the value of 100. The moisture resistance is indicated by the relative value of the residual density after the preservation for three weeks under the conditions at the temperature of 60°C and at the relative humidity of 70% to the value of the density measured before testing that is regarded as the value of 100. Further, the

heat resistance is indicated by the relative value of the residual density after the preservation for three weeks under the conditions at the temperature of 77°C to the value of the density measured before testing that is regarded as the value of 100, provided that the initial density is taken at 1.0.

As is obvious from Table 2, the control coupler [A] has the problem in the heat-and-moisture resistance while it has the excellent performance in the light resistance, and the control couplers [B] and [C] have the problem in the light resistance in the process of the color development [2] while they are excellent in the heat-and-moisture resistance.

On the other hand, it is found that the exemplified couplers [1], [3] and [8] relating to the invention are the couplers having the excellent performance in every aspect.

Example (3)

There were taken 0.01 mol each of the couplers of the invention indicated in Table 3, the aforesaid Control Couplers [A] and [B] and the under-mentioned Control Coupler [D], and the addition thereto were made respectively with the mixture solution of tricresyl phosphate whose amount is equivalent to the weight of said coupler added and ethyl acetate whose amount is three times as much as the weight of said coupler added, and then the dissolution was completed by raising the temperature up to 60°C. The solution thus prepared was added into an

aqueous solution of Alkanol B and gelatin and the emulsification was made by making use of a colloid-mill, and thus, the coupler-dispersion solutions were prepared, respectively.

Next, each of said coupler-dispersion solutions was added into silver iodobromide emulsion (6 mol% thereof were silver iodide) containing 0.1 mol in silver equivalent, and the solution thus obtained was coated on a sheet of cellulose acetate film-base, and the drying was then made. Thus, there have obtained the six kinds of silver halide color photographic materials (Sample Nos. [13] through [18]) having the stable coated layer.

Control Coupler [D]

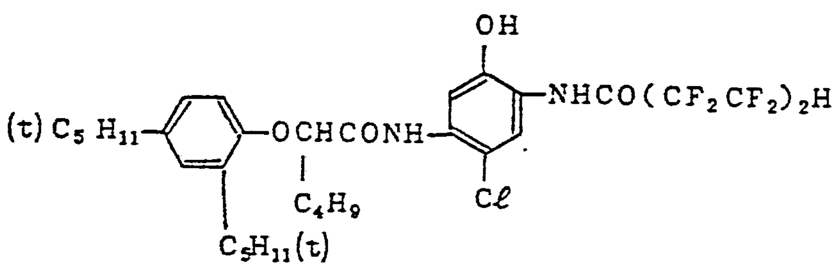

Each of these samples was exposed to light through a wedge in a usual process, and the following process was then applied.

[Process]

| Processing Steps (at 33°C) | Processing time |
|---|---|
| Color development | 3' 15" |
| Bleaching | 6' 30" |
| Washing | 3' 15" |

| | |
|---|---|
| Fixing | 6' 30" |
| Washing | 3' 15" |
| Stabilizing | 1' 30" |

[Composition of Color Developing Solution]

| | |
|---|---|
| 4-amino-3-methyl-N-ethyl-N-(β-hydroxy ethyl)-aniline sulfate | 4.8 g |
| Sodium sulfite, anhydrous | 0.14 g |
| Hydroxyamine, 1/8 sulfate | 1.98 g |
| Sulfuric acid | 0.74 g |
| Potassium carbonate, anhydrous | 28.85 g |
| Potassium hydrogencarbonate, anhydrous | 3.46 g |
| Potassium sulfite, anhydrous | 5.10 g |
| Potassium bromide | 1.16 g |
| Sodium chloride | 0.14 g |
| Trisodium nitriloacetate | 1.20 g |
| Potassium hydroxide | 1.48 g |
| Add water to make | 1 ltr. |

[Composition of Bleaching Solution]

| | |
|---|---|
| Ammonium iron ethylenediamine tetraacetate | 100 g |
| Diammonium ethylenediamine tetraacetate | 10 g |
| Ammonium bromide | 150 g |
| Glacial acetic acid | 10 ml |
| Add water to make | 1 ltr. |
| Adjust pH value with aqueous ammonia to | pH 6.0 |

[Composition of Fixing Solution]

| | |
|---|---|
| Ammonia thiosulfate | 175.5 g |
| Sodium sulfite, anhydrous | 8.6 g |
| Sodium metasulfite | 2.3 g |
| Add water to make | 1 ltr. |
| Adjust pH value with acetic acid to | pH 6.0 |

[Composition of Stabilizing Solution]

| | |
|---|---|
| Formalin (37% aqueous solution) | 1.5 ml |
| Konidux (mfd. by Konishiroku Photo Ind. Co., Ltd.) | 7.5 ml |
| Add water to make | 1 ltr. |

Thus obtained cyan color image was measured for the photographic characteristics. The results therefrom are shown in Table 3.

Table 3

| Sample No. | Coupler used | Relative Sensitivity | Max. Density |
|---|---|---|---|
| 13 | Exemplified Compound-10 | 97 | 2.15 |
| 14 | " -15 | 100 | 2.26 |
| 15 | " -17 | 98 | 2.22 |
| 16 | Control Coupler - A | 98 | 1.67 |
| 17 | " - B | 92 | 1.70 |
| 18 | " - D | 80 | 1.55 |

As is obvious from Table 3, it can be found that the samples having used the couplers relating to the invention are excellent in the sensitivity and the color developability.

It was also found from the results of measuring the spectrum of the samples prepared through the invention that said samples have the maximum absorption wavelength in the long wave band of red spectrum area and display the sharp-cut resolving power in the short wavelength band and further endow with the desirable color dye images for the color reproduction of the green area.

Example (4)

Samples (1) through (6) prepared in Example-1 were exposed to light through a wedge and were then processed to develop them as in Example-1.

On the other hand, the development process was carried out with the bleach-fix solution of which the composition replaced by the following composition, and the color fading property of cyan dye was investigated with a fatigued bleach-fix solution.

[Composition of Bleach-Fix Solution]

| | |
|---|---|
| Ammonium iron ethylenediamine-tetraacetate | 50 g |
| Ammonium sulfite (40% solution) | 50 ml |
| Ammonium thiosulfate (70% solution) | 140 ml |
| Aqueous ammonia (28% solution) | 20 ml |

| | |
|---|---|
| Ethylenediamine tetraacetic acid | 4 g |
| Hydrosulfite | 5 g |
| Add water to make | 1 ltr. |

Measurements were made for the maximum reflection density of the cyan dye in the samples prepared in the development process. The results therefrom are shown in Table 4.

Wherein, the rate of residual dyes in the maximum density area was formularized as follows:

$$\text{Rate of Residual Dye} = \frac{\text{Process with Fatigued Bleach-Fix Solution}}{\text{Process with Fresh Bleach-Fix Solution}} \times 100$$

Table 4

| Coupler used | Fresh Bleach Fix Process | Fatigued Bleach Fix Process | Rate of Residual Dye |
|---|---|---|---|
| Exemplified Compound - 1 | 2.24 | 2.22 | 99 |
| " - 3 | 2.28 | 2.25 | 99 |
| " - 8 | 2.19 | 2.17 | 99 |
| Control Coupler - A | 2.17 | 1.40 | 65 |
| " - B | 1.90 | 1.86 | 98 |
| " - C | 1.82 | 1.75 | 96 |

It is understandable from Table 4 that the samples having used the couplers relating to the invention are less in the cyan dye fading in the processing with the fatigued bleach-fix solution.

Example (5)

There were taken 0.03 mol each of the couplers of the invention indicated in Table 5 and the undermentioned control couplers, (A), (B) and (C) to add respectively into each of the mixed solutions of dibutyl phthalate whose amount was equivalent to the weight of said coupler added and ethyl acetate whose amount was three times as much as the weight of said coupler added, and then the dissolution was completed by raising the temperature up to 60°C. And each of the coupler dispersion solution was prepared in the process that the solution thus obtained was added into the aqueous solution of Alkanol B (alkylnaphthalene sulfonate - mfd. by Du Pont) and gelatin, and the emulsification was made by means of a colloid-mill. Next, each of said coupler dispersion solutions was added into silver chlorobromide emulsion of 0.1 mol in silver equivalent (20 mol% thereof were silver bromide) and thus obtained solution was coated on a sheet of polyethylene laminated paper and was then dried up, and thus, six kinds, in all, of the silver halide color photographic materials (Sample No. [19] - [24]) having the stably coated layer were obtained, respectively.

Control Coupler [A]

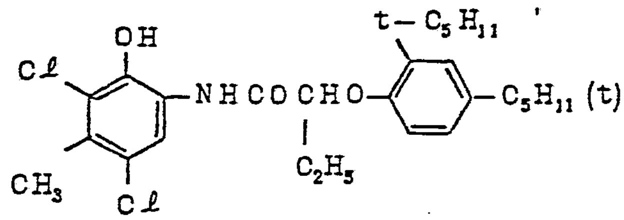

Control Coupler [B]

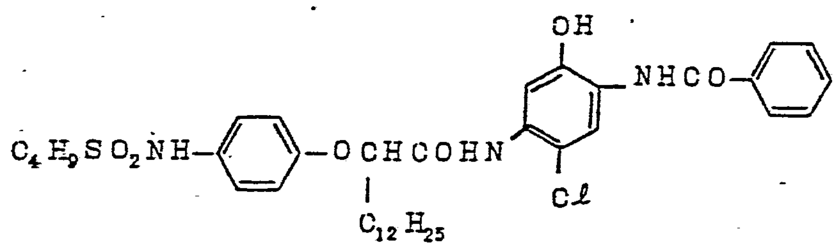

Control Coupler [C]

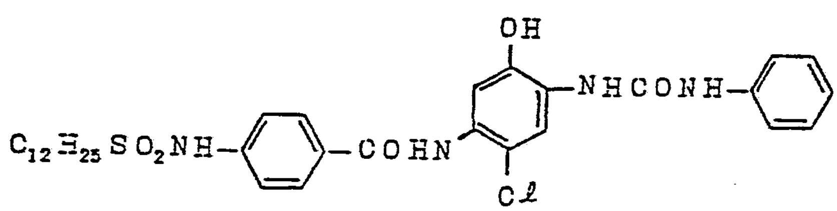

Each of these samples was exposed to light through a wedge and then processed as mentioned hereunder, provided that the color developing processes were carried out respectively on the two kinds of the compositions, i.e.; the composite added with benzyl alcohol (Composition of color developing solution [1]) and the composite without addition thereof (Composition of color development [2]).

[Process]

| Processing Steps (at 30°C) | Processing Time |
|---|---|
| Color development | 3' 30" |
| Bleach-Fix | 1' 30" |
| Wash | 2' |

The following shows each of the processing compositions:

[Composition of Color Developing Solution 1]

| | |
|---|---|
| 4-amino-3-methyl-N-ethyl-N-(β-methane sulfonamide ethyl)-aniline sulfate | 5.0 g |
| Benzyl alcohol | 15.0 ml |
| Sodium hexametaphosphate | 2.5 g |
| Sodium sulfite, anhydrous | 1.85 g |
| Sodium bromide | 1.4 g |
| Potassium bromide | 0.5 g |
| Borax | 39.1 g |
| Add water to make | 1.0 ltr. |
| Adjust pH value by sodium hydroxide to pH 10.30 | |

[Composition of Color Developing Solution 2]

| | |
|---|---|
| 4-amino-3-methyl-N-ethyl-N-(β-methane sulfonamide ethyl)-aniline sulfate | 5.0 g |
| Sodium hexametaphosphate | 2.5 g |
| Sodium sulfite, anhydrous | 1.85 g |
| Sodium bromide | 1.4 g |
| Potassium bromide | 0.5 g |
| Borax | 39.1 g |

| | |
|---|---|
| Add water to make | 1.0 ltr. |
| Adjust pH value by sodium hydroxide to pH 10.30 | |

[Composition of Bleach-Fix Solution]

| | |
|---|---|
| Iron ethylenediamine tetraacetate ammonium salt | 50 g |
| Ammonium sulfite (40% solution) | 50 ml |
| Ammonium thiosulfate (70% solution) | 140 ml |
| Aqueous ammonia (28% solution) | 20 ml |
| Ethylenediamine tetraacetate | 4 g |
| Add water to make | 1 ltr. |

The photographic characteristics of each sample thus obtained were measured. The results thereof are tabulated in Table 5.

Wherein, the values of the relative sensitivity are indicated relatively to the maximum value of sensitivity, that is regarded as the value of 100, obtained when the processing was made with Color developing solution (1).

Table 5

| Sample No. | Coupler used | Color Development (1) | | Color Development (2) | |
|---|---|---|---|---|---|
| | | Relative Sensitivity | Max. Density | Relative Sensitivity | Max. Density |
| 19 | Exemplified Compound-20 | 100 | 2.30 | 74 | 1.90 |
| 20 | " -24 | 97 | 2.18 | 70 | 1.81 |
| 21 | " -25 | 99 | 2.25 | 72 | 1.85 |
| 22 | Control Coupler - A | 95 | 2.16 | 50 | 1.40 |
| 23 | " - B | 92 | 1.87 | 62 | 1.51 |
| 24 | " - C | 84 | 1.85 | 59 | 1.50 |

It is obvious from the above Table 5, it is found that the samples obtained by using the couplers of the invention are capable of obtaining the excellent sensitivity and maximum density with or without addition of benzyl alcohol.

Also, as the result of measuring the chromatic emission spectrum, it was found that the dyes having used the couplers of the invention had the absorption maximum in the portion of the relatively longer wavelength in the red area and had a less absorption on the short wavelength side, so that an excellent color purity displayed.

Example (6)

The light resistance, heat resistance and moisture resistance of a color dye image were studied on the samples prepared in the similar process to that taken in Example (5).

The results therefrom are tabulated in Table 6.

Table 6

| Sample No. | Coupler used | Color Development (1) | | | Color Development (2) | | |
|---|---|---|---|---|---|---|---|
| | | Light Resistance | Heat Resistance | Moisture Resistance | Light Resistance | Heat Resistance | Moisture Resistance |
| 25 | Exemplified Compound-20 | 92 | 98 | 98 | 90 | 99 | 98 |
| 26 | " -24 | 86 | 94 | 97 | 84 | 96 | 98 |
| 27 | " -25 | 85 | 95 | 97 | 87 | 96 | 97 |
| 28 | Control Coupler - A | 85 | 45 | 60 | 86 | 46 | 62 |
| 29 | " - B | 62 | 95 | 93 | 57 | 95 | 95 |
| 30 | " - C | 55 | 92 | 92 | 52 | 94 | 93 |

In the above Table 6, the value of the light resistance of each coupler is indicated by the relative value of the residual density of each image exposed by a xenon fade meter for 300 hours to the value of the density thereof measured before the exposure that is regarded as the value of 100. The moisture resistance is indicated by the relative value of the residual

density after the preservation for three weeks under the conditions at the temperature of 60°C and at the relative humidity of 70% to the value of the density measured before testing that is regarded as the value of 100. Further, the heat resistance is indicated by the relative value of the residual density after the preservation for three weeks under the conditions at the temperature of 77°C to the value of the density measured before testing that is regarded as the value of 100, provided that the initial density is taken at 1.0.

As is obvious from Table 6, the control coupler [A] has the problem in the heat-and-moisture resistance while it has the excellent performance in the light resistance, and the control couplers [B] and [C] have the problem in the light resistance in the process of the color development [2] while they are excellent in the heat-and-moisture resistance.

On the other hand, it is found that the exemplified couplers [20], [24] and [25] relating to the invention are the couplers having the excellent performance in every aspect.

Example (7)

there were taken 0.01 mol each of the couplers of the invention indicated in Table 7, the aforesaid Control Coupler [A] and [B] and the under-mentioned Control Coupler [D], and the addition thereto were made respectively with the mixture solution of tricresyl phosphate whose amount is equivalent to

the weight of said coupler added and ethyl acetate whose amount is three times as much as the weight of said coupler added, and then the dissolution was completed by raising the temperature up to 60°C. The solution thus prepared was added into an aqueous solution of Alkanol B and gelatin and the emulsification was made by making use of a colloid-mill, and thus, the coupler-dispersion solutions were prepared, respectively.

Next, each of said coupler-dispersion solutions was added into silver iodobromide emulsion (6 mol% thereof were silver iodide) containing 0.1 mol in silver equivalent, and the solution thus obtained was coated on a sheet of cellulose acetate film-base, and the drying was then made. Thus, there have obtained the six kinds of silver halide color photographic materials (Sample Nos. [31] through [36] having the stable coated layer.

Control Coupler [D]

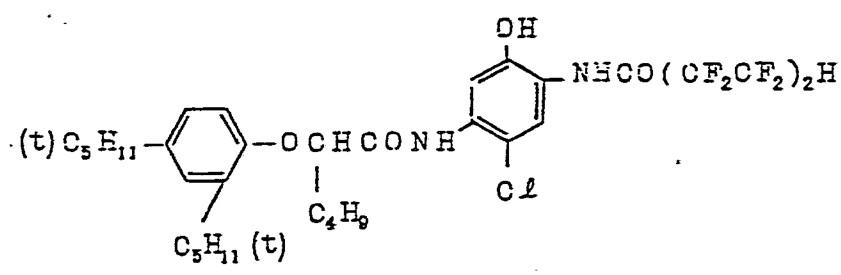

Each of these samples was exposed to light through a wedge in a usual process, and the following process was then applied.

[Process]

| Processing Steps (at 33°C) | Processing Time |
|---|---|
| Color development | 3' 15" |
| Bleaching | 6' 30" |
| Washing | 3' 15" |
| Fixing | 6' 30" |
| Washing | 3' 15" |
| Stabilizing | 1' 30" |

[Composition of Color Developing Solution]

| | |
|---|---|
| 4-amino-3-methyl-N-ethyl-N-(β-hydroxy ethyl)-aniline sulfate | 4.8 g |
| Sodium sulfite, anhydrous | 0.14 g |
| Hydroxyamine, 1/8 sulfate | 1.98 g |
| Sulfuric acid | 0.74 g |
| Potassium carbonate, anhydrous | 28.85 g |
| Potassium hydrogencarbonate, anhydrous | 3.46 g |
| Potassium sulfite, anhydrous | 5.10 g |
| Potassium bromide | 1.16 g |
| Sodium chloride | 0.14 g |
| Trisodium nitriloacetate | 1.20 g |
| Potassium hydroxide | 1.48 g |
| Add water to make | 1 ltr. |

[Composition of Bleaching Solution]

| | |
|---|---|
| Ammonium iron ethylenediamine tetraacetate | 100 g |

| | |
|---|---|
| Diammonium ethylenediamine tetraacetate | 10 g |
| Ammonium bromide | 150 g |
| Glacial acetic acid | 10 ml |
| Add water to make | 1 ltr. |
| Adjust pH value with aqueous ammonia to | pH 6.0 |

[Composition of Fixing Solution]

| | |
|---|---|
| Ammonia thiosulfate | 175.5 g |
| Sodium sulfite, anhydrous | 8.6 g |
| Sodium metasulfite | 2.3 g |
| Add water to make | 1 ltr. |
| Adjust pH value with acetic acid to | pH 6.0 |

[Composition of Stabilizing Solution]

| | |
|---|---|
| Formalin (37% aqueous solution) | 1.5 ml |
| Konidux (mfd. by Konishiroku Photo Ind. Co., Ltd.) | 7.5 ml |
| Add water to make | 1 ltr. |

Thus obtained cyan color image was measured for the photographic characteristics. The results therefrom are shown in Table 7.

Table 7

| Sample No. | Coupler used | Relative Sensitivity | Max. Density |
|---|---|---|---|
| 31 | Exemplified Compound-29 | 100 | 2.33 |
| 32 | " -34 | 96 | 2.16 |
| 33 | " -41 | 98 | 2.18 |
| 34 | Control Coupler -A | 96 | 1.65 |
| 35 | " -B | 90 | 1.70 |
| 36 | " -D | 80 | 1.52 |

As is obvious from Table 7, it can be found that the samples having used the couplers relating to the invention are excellent in the sensitivity and the color developability.

It was also found from the results of measuring the spectrum of the samples prepared through the invention that said samples have the maximum absorption wavelength in the long wave band of red spectrum area and display the sharp-cut resolving power in the short wavelength band and further endow with the desirable color dye images for the color reproduction of the green area.

Example (8)

Samples (19) through (24) prepared in Example-5 were exposed

to light through a wedge and were then processed to develop them as in Example-5.

On the other hand, the development process was carried out with the bleach-fix solution of which the composition replaced by the following composition, and the color fading property of cyan dye was investigated with a fatigued bleach-fix solution.

[Composition of Bleach-Fix Solution]

| | |
|---|---|
| Ammonium iron ethylenediamine-tetraacetate | 50 g |
| Ammonium sulfite (40% solution) | 50 ml |
| Ammonium thiosulfate (70% solution) | 140 ml |
| Aqueous ammonia (28% solution) | 20 ml |
| Ethylenediamine tetraacetic acid | 4 g |
| Hydrosulfite | 5 g |
| Add water to make | 1 ltr. |

Measurements were made for the maximum reflection density of the cyan dye in the samples prepared in the development process. The results therefrom are shown in Table 8.

Wherein, the rate of residual dyes in the maximum density area obtained was formularized as follows:

$$\text{Rate of Residual Dye} = \frac{\text{Process with Fatigued Bleach-Fix Solution}}{\text{Process with Fresh Bleach-Fix Solution}} \times 100$$

Table 8

| Coupler used | Fresh Bleach Fix Process | Fatigued Bleach Fix Process | Rate of Residual Dye |
|---|---|---|---|
| Exemplified Compound-20 | 2.30 | 2.28 | 99 |
| " -24 | 2.18 | 2.16 | 99 |
| " -25 | 2.25 | 2.20 | 98 |
| Control Coupler - A | 2.16 | 1.42 | 66 |
| " - B | 1.87 | 1.84 | 98 |
| " - C | 1.85 | 1.77 | 96 |

It is understandable from Table 8 that the samples having used the couplers relating to the invention are less in the cyan dye fading in the processing with the fatigued bleach-fix solution.

CLAIMS

1. A phenol cyan coupler suitable for use in a silver halide color photographic material comprising a phenol nucleus substituted by a group represented by the following Formula [I] or [II] at the 2-position and by an acylamino group at the 5-position:

Formula [I]

$$-NHCONH-R_1$$

wherein $R_1$ is a heterocyclic group or a condensed heterocyclic group,

Formula [II]

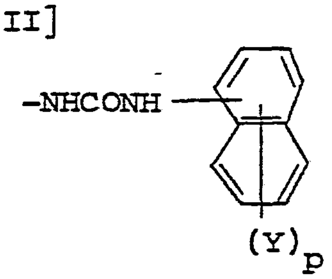

wherein Y is a monovalent group; p is o or an integer from one to five.

2. A cyan coupler according to claim 1, which is represented by the following Formula [III] or [IV]:

Formula [III]

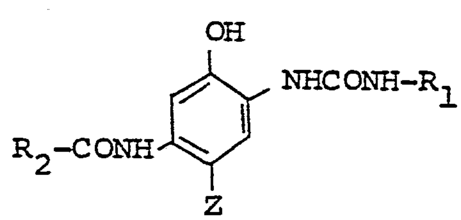

wherein $R_1$ is as defined in claim 1, $R_2$ is a ballast group and Z is a hydrogen atom or a group removable upon coupling with an oxidation product of a color

developing agent,

Formula [IV]

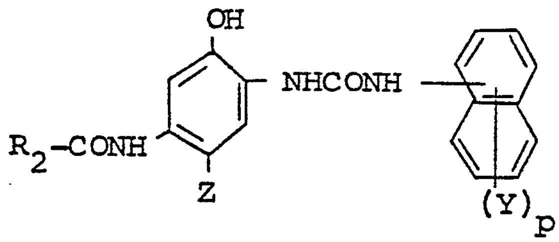

wherein Y and p are as defined in claim 1 and $R_2$ and Z are as defined above in connection with Formula [III].

3. A cyan coupler according to claim 2, wherein the ballast group represented by $R_2$ in Formula [III] or [IV] is an alkyl, an aralkyl, an alkenyl, an aralkenyl, an alkoxyalkyl, a cycloalkyl or a five or six membered hetero-cyclic group and a group represented by the following Formula [V]:

Formula [V]

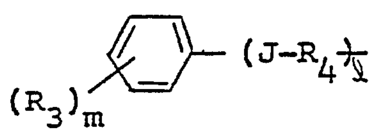

wherein $R_3$ is a halogen atom, an alkyl, an aryl, a heterocyclic, an aralkyl, an alkoxy, a carboxy, an alkoxycarbonyl, an aryloxycarbonyl, a mercapto, an alkylthio, an acyl, an acylamino, a sulfonamido, a carbamoyl or sulfamoyl group, $R_4$ is an alkyl group having 1 - 20 carbon atoms, J is an oxygen atom or a sulfur atom, m is an integer from 1 to 4, and $\ell$ is 0 or 1.

4. A cyano coupler according to claim 2 or 3, wherein the removable group represented by Z in Formula [III] or [IV] is an aryloxy group, a carbamoyloxy group, a carbamoylmethoxy group, an acyloxy group, a sulfonamide group or a succinimide group.

5. A cyan coupler according to any one of claims 2 to 4, wherein the monovalent group represented by Y in Formula [II] or [IV] is an alkyl group, an aryl group, a heterocyclic group, a hydroxy group, an alkoxy group, an aryloxy group, an acyloxy group, a mercapto group, an alkylthio group, a nitro group, an acyl group, an amino group, an alkylamino group, a dialkylamino group, a halogen atom, a cyano group or a group of formula $-COOR_5$, $-CON(R_5)_2$, $-SO_2OR_6$, $-SO_2N(R_5)_2$ or $-SOR_6$, wherein $R_6$ is an alkyl group, an alkenyl group, a cycloalkyl group, an aryl group or a heterocyclic group, each $R_5$ independently is as defined under $R_6$ such that $R_5$ and $R_6$ may be identical or different or both $R_5$ radicals together form, with the nitrogen atom to which they are attached, a five- or six-membered ring.

6. A silver halide photographic material which comprises a phenol cyan coupler as claimed in any one of the preceding claims.

7. A material according to claim 6 in which the coupler is present in a silver halide emulsion layer.

European Patent Office

# EUROPEAN SEARCH REPORT

0073145

Application number

EP 82 30 4385

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A,D | FR-A-1 379 850 (AGFA)<br>* compound XV * & GB - A - 1 011 940<br>----- | | G 03 C 7/34 //<br>C 07 D 213/75<br>C 07 D 307/66<br>C 07 C 127/19 |
| | | | **TECHNICAL FIELDS SEARCHED (Int. Cl. 3)** |
| | | | G 03 C 7/00<br>C 07 C 127/00 |

The present search report has been drawn up for all claims

| Place of search | Date of completion of the search | Examiner |
|---|---|---|
| THE HAGUE | 28-09-1982 | PHILOSOPH L.P. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document